# FASCICULE DE BREVET EUROPEEN

(11) **EP 3 576 830 B1**
(45) Date de publication et mention de la délivrance du brevet: **30.06.2021**
(21) Numéro de dépôt: 18705334.3
(22) Date de dépôt: 01.02.2018
(51) Int. Cl.: A61M 27/00

(54) **DISPOSITIF DE REGLAGE D'UNE VALVE DE DRAINAGE**
VORRICHTUNG ZUM EINSTELLEN EINES DRAINAGEVENTILS
DEVICE FOR ADJUSTING A DRAINAGE VALVE

(30) Priorité: 01.02.2017 FR 1750840
(43) Date de publication de la demande: 11.12.2019
(73) Titulaire: Sophysa, 91400 Orsay (FR)
(72) Inventeur: NEGRE, Philippe, 75116 Paris (FR)
(74) Mandataire: Nony
(86) Numéro de dépôt international: PCT/EP2018/052544
(87) Numéro de publication internationale: WO 2018/141859

(56) Documents cités:
- EP-A1- 2 236 169
- EP-A1- 2 316 522
- EP-A1- 2 865 408
- EP-A2- 2 420 284
- EP-A2- 2 777 751

## Description

### Domaine technique

La présente invention a pour objet un dispositif de traitement, notamment de réglage d'une valve de drainage destinée à des applications thérapeutiques, notamment le traitement de l'hydrocéphalie.

### Etat de la technique

Le traitement de l'hydrocéphalie consiste à dériver du liquide céphalo-rachidien (LCR) contenu dans les ventricules cérébraux ou l'espace sous-arachnoïdien lombaire vers un site de résorption, par exemple la cavité péritonéale.

Le brevet européen EP 688 575 de la déposante divulgue une valve adaptée pour ce traitement. En particulier, la valve comporte
- un corps définissant une chambre interne sensiblement cylindrique et plate,
- un conduit d'entrée et un conduit d'évacuation ménagés dans la paroi latérale de ladite chambre et aptes à être respectivement connectés à un cathéter d'amenée et un cathéter de drainage de liquide,
- un rotor apte à tourner dans ladite chambre autour de son axe central,
- un obturateur, appelé « clapet anti-retour », tel une bille, disposé au niveau de l'extrémité interne du conduit d'entrée,
- un organe de rappel élastique, en l'occurrence un ressort à lame courbe, de préférence semi-circulaire, fixé audit rotor, parallèle à la paroi latérale de la chambre et comprimant le clapet dans son siège de manière à réguler, et le cas échéant bloquer le passage de liquide dans la chambre par le conduit d'entrée.

La rotation du rotor entraîne le glissement du point de contact de la bille sur le ressort à lame, et donc une modification de la pression d'ouverture.

La valve de EP 688 575 comporte deux micro-aimants montés dans le rotor et disposés de part et d'autre de l'axe central de la chambre. Ces deux micro-aimants peuvent être couplés magnétiquement à une clé magnétique, appelée « dispositif de réglage externe de la valve » dans EP 688 575, manipulée par un opérateur. La clé magnétique comporte un barreau magnétique constitué d'une combinaison d'aimants. La clé magnétique permet ainsi, depuis l'extérieur, à travers des tissus cutanés, de modifier la position angulaire du rotor, et donc la pression minimale permettant au liquide de déplacer le clapet pour dégager le conduit d'entrée, appelée « pression d'ouverture ».

Les micro-aimants du rotor sont mobiles linéairement dans ledit rotor selon une direction sensiblement radiale de celui-ci. En l'absence de la clé, les deux micro-aimants sont maintenus, par exemple par attraction réciproque, dans une position de verrouillage du rotor. En particulier, les deux micro-aimants peuvent maintenir des ergots respectifs dans des cavités ménagées circulairement dans la chambre. Sous l'effet magnétique de la clé, les deux micro-aimants se déplacent jusqu'à une position de déverrouillage du rotor, par exemple en extrayant lesdits ergots desdites cavités.

La même clé peut être avantageusement utilisée pour déverrouiller le rotor, puis pour l'entrainer en rotation.

En pratique, l'inventeur a constaté que l'entraînement du rotor n'est pas toujours fiable. Par ailleurs, le déverrouillage peut être laborieux, même avec une clé magnétique de forte puissance. Il existe donc un besoin pour un procédé et un dispositif de réglage qui soient plus fiables.

EP 2 236 169 décrit un dispositif de lecture et de réglage d'une valve implantable.

EP 2 865 408 décrit un outil réglable en hauteur pour localiser une valve implantable, et lire et régler les paramètres de la valve magnétiquement.

Un objectif de la présente invention est de répondre, au moins partiellement, à ce besoin.

L'invention est définie par un dispositif comme dans la revendication 1. Des aspects additionnels sont définis dans les revendications dépendantes 2 à 12.

### Résumé de l'invention

On décrit également un procédé de réglage de la pression d'ouverture d'une valve implantable, notamment destinée au traitement de l'hydrocéphalie, en particulier d'une valve décrite dans EP 688 575 ou dans EP 3 046 614 , ladite valve comportant un rotor d'axe de rotation R dont la position angulaire détermine ladite pression d'ouverture, ledit rotor comportant un micro-aimant de rotor mobile entre des positions de verrouillage et de déverrouillage dudit rotor, dans lesquelles ledit micro-aimant interdit et autorise la rotation dudit rotor, respectivement, le procédé comprenant les étapes successives suivantes :
a) disposition d'un localisateur de manière à aligner un axe X d'un logement de localisateur ménagé dans le localisateur avec l'axe de rotation R du rotor et à orienter le localisateur dans une position angulaire prédéfinie autour de l'axe de rotation R ;
b) détermination de la position angulaire du rotor autour de son axe de rotation R, par rapport au localisateur ;
c) insertion d'un actionneur comportant un aimant dans le logement de localisateur, selon l'axe X, jusqu'à une première profondeur d'insertion;
d) retrait partiel dudit actionneur du logement de localisateur, selon l'axe X, jusqu'à une deuxième profondeur d'insertion inférieure à la première profondeur d'insertion ;
e) rotation dudit actionneur à partir de la deuxième profondeur d'insertion, de préférence en maintenant ledit actionneur à ladite deuxième profondeur d'insertion ;
f) écartement de l'actionneur de la valve, de préférence par extraction complète de l'actionneur hors du logement de localisateur.

De manière surprenante, l'inventeur a découvert que, selon la profondeur d'insertion, l'effet de couplage magnétique entre le micro-aimant du rotor et l'aimant de l'actionneur est plus favorable au déverrouillage ou à l'entraînement du rotor. En particulier, la première profondeur d'insertion est particulièrement favorable au déverrouillage alors que la deuxième profondeur d'insertion est plus favorable à l'entraînement du rotor. En pratique, il suffit donc à l'opérateur d'appuyer sur l'actionneur pour déverrouiller, puis de le retirer partiellement avant de le tourner pour régler la pression d'ouverture.

De préférence, à l'étape a), pour atteindre ladite position angulaire prédéfinie, on dispose ladite valve dans une fenêtre du localisateur, ladite fenêtre étant conformée de manière que, lorsque ladite valve y est logée, des axes d'orientation O_{V} et O_{L} de la valve et de ladite fenêtre soient sensiblement parallèles l'un à l'autre et que l'axe de rotation R du rotor de la valve et l'axe X soient sensiblement confondus.

Dans un mode de réalisation, le procédé est utilisé pour régler une valve implantée sous la peau d'un patient.

L'invention concerne un dispositif de réglage de la pression d'ouverture d'une valve implantable, notamment pour le traitement de l'hydrocéphalie, comme dans la revendication 1. Le dispositif comporte :
- un localisateur de valve définissant un logement de localisateur d'axe X ;
- une clé comportant un actionneur comportant un aimant d'actionneur, l'actionneur étant insérable, selon l'axe X, dans le logement de localisateur à une première profondeur d'insertion et à une deuxième profondeur d'insertion inférieure à la première profondeur d'insertion ;
le dispositif comportant un guide de la rotation de l'actionneur autour de l'axe X depuis au moins une position angulaire de l'actionneur autour de l'axe X, de préférence depuis n'importe quelle position angulaire de l'actionneur autour de l'axe X, dans laquelle l'actionneur est à la deuxième profondeur d'insertion, de préférence dans un plan de rotation sensiblement perpendiculaire à l'axe X.

Ainsi, à partir d'au moins une position angulaire de l'actionneur, l'opérateur peut faire tourner ledit actionneur autour de l'axe X de manière guidée. Le mouvement de l'actionneur lors de cette rotation n'est pas nécessairement une simple rotation autour de l'axe X, mais peut se combiner, par exemple, avec une translation selon l'axe X et/ou une autre rotation. De préférence cependant, le mouvement de l'actionneur lors de cette rotation est une simple rotation autour de l'axe X.

Par « guidage », on entend que l'opérateur qui se contente de tourner l'actionneur autour de l'axe X donne à l'actionneur un mouvement prédéterminé. L'opérateur peut cependant quitter le guidage. Par exemple, dans le mode de réalisation décrit en détail ci-après, il peut appuyer sur l'actionneur pour l'enfoncer dans le logement de localisateur.

Un dispositif selon l'invention peut être en particulier utilisé pour la mise en œuvre d'un dit procédé de réglage.

Suivent ici quelques exemples de caractéristiques optionnelles :
- le dispositif comporte un guide de la rotation de la clé autour de l'axe X depuis au moins une position angulaire de l'actionneur autour de l'axe X, de préférence depuis n'importe quelle position angulaire de l'actionneur autour de l'axe X, à la première profondeur d'insertion, de préférence dans un plan de rotation perpendiculaire à l'axe X;
- La différence entre les première et deuxième profondeurs d'insertion, mesurées selon l'axe X, est supérieure à 2 mm, 3 mm, 4 mm, 5 mm et/ou inférieure à 15 mm, 12 mm, 10 mm, 8 mm, 7 mm, ou 6 mm ;
- La clé comporte un rehausseur sur lequel l'actionneur est monté mobile en translation selon un axe Y de la clé qui, lorsque la clé est insérée dans le logement de localisateur, se confond avec l'axe X du logement de localisateur ;
- L'actionneur est guidé en translation sur le rehausseur, selon l'axe Y ;
- Le dispositif comporte une première butée, ou « butée vers le bas » ou « butée basse », limitant l'insertion de l'actionneur dans le logement de localisateur, de préférence quelle que soit la position angulaire de l'actionneur autour de l'axe X, ladite première butée définissant la première profondeur d'insertion et étant de préférence constituée par un fond du logement de localisateur ou par un fond du rehausseur de la clé ;
- Le dispositif comporte une deuxième butée, ou « butée vers le haut », ou « butée haute », définissant la deuxième profondeur d'insertion et limitant l'extraction de l'actionneur hors du logement de localisateur et/ou guidant la rotation de l'actionneur à la deuxième profondeur d'insertion ;
- Ladite deuxième butée constitue un dit guide de la rotation de l'actionneur autour de l'axe X ;
- De préférence, le dispositif comporte un ressort, de préférence encore escamotable, compressible sous l'effet d'une poussée d'insertion de l'actionneur dans le logement de localisateur et maintenant l'actionneur à la deuxième profondeur d'insertion en l'absence de ladite poussée ;
- Ledit ressort s'étend de préférence selon un axe sensiblement parallèle à l'axe X et/ou à l'axe Y ;
- De préférence, la première butée et/ou la deuxième butée sont conformées de manière à être actives indépendamment de la position angulaire de l'actionneur autour de l'axe X;
- De préférence, la clé comporte un rehausseur sur lequel l'actionneur est mobile selon l'axe Y et ledit ressort est interposé entre l'actionneur et le rehausseur ou entre l'actionneur et le localisateur, de manière à s'opposer à une insertion de l'actionneur, selon l'axe Y, au-delà de la deuxième profondeur d'insertion ;
- l'actionneur comporte un ensemble d'aimants agencés suivant une configuration de Halbach ;
- l'actionneur comporte un support sur lequel ledit aimant d'actionneur est rigidement fixé, une bride montée mobile en translation, selon l'axe Y, par rapport au rehausseur et des moyens pour empêcher tout déplacement du support selon l'axe Y, par rapport à la bride, tout en autorisant un déplacement du support perpendiculairement à l'axe Y, par rapport à la bride.

Les caractéristiques décrites ci-dessus peuvent être combinées entre elles ou avec une ou plusieurs des caractéristiques ci-dessous.
- On décrit également un dispositif de traitement comportant
- une valve implantable comportant :
   - un corps définissant une chambre interne,
   - un conduit d'entrée et un conduit d'évacuation ménagés dans la paroi latérale de ladite chambre interne et aptes à être respectivement connectés à un cathéter d'amenée et un cathéter de drainage de liquide,
   - un rotor apte à tourner dans ladite chambre interne autour d'un axe de rotation R, le rotor comportant un micro-aimant mobile entre des positions de verrouillage et de déverrouillage du rotor, dans lesquelles il interdit et autorise la rotation dudit rotor, respectivement,
   - un obturateur,
   - un organe de rappel élastique comprimant l'obturateur dans son siège de manière à réguler, et le cas échéant bloquer le passage de liquide dans la chambre interne, et
- un dispositif de réglage de la pression d'ouverture de ladite valve, le dispositif de réglage étant selon l'invention.

La valve peut en particulier comporter une ou plusieurs des caractéristiques des valves décrites dans EP 688 575 ou dans EP 3 046 614. En particulier, de préférence, elle comporte exactement deux micro-aimants montés dans le rotor et disposés de part et d'autre de l'axe du rotor.

Une rotation du rotor, de préférence toute rotation du rotor modifie la compression de l'obturateur par l'organe de rappel élastique, et donc modifie la pression d'ouverture de la valve.

De préférence, le rotor n'est mobile qu'en rotation autour de l'axe de rotation. En particulier, il n'est pas mobile en translation le long de cet axe.

Dans un dispositif selon l'invention, l'aimant d'actionneur exerce un champ magnétique adapté pour, lorsque le localisateur est disposé contre la peau d'un patient sous laquelle la valve a été implantée, la valve étant disposée dans la fenêtre du localisateur, déverrouiller le rotor à la première profondeur d'insertion et être couplé en rotation avec le rotor à la deuxième profondeur d'insertion.

De préférence, l'aimant d'actionneur est configuré pour exercer un champ magnétique adapté pour, lorsque la valve est à l'air libre et que le localisateur est disposé perpendiculairement à l'axe de rotation du rotor de la valve,
- ne déverrouiller le rotor que lorsque l'aimant d'actionneur est à une distance du rotor inférieure à 20 mm, 15 mm, 12 mm, voire 10 mm ; et/ou
- n'être couplé en rotation avec le rotor que lorsque l'aimant d'actionneur est à une distance du rotor inférieure à 30 mm, 25 mm, 20 mm, 18 mm, ou 16 mm.

### Définitions

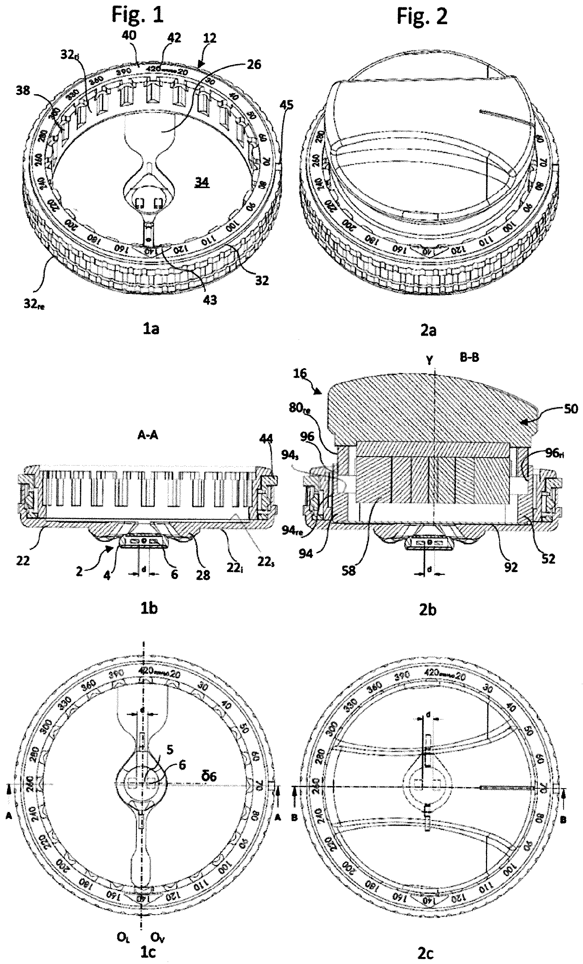

- Les adjectifs « supérieur », « inférieur », « bas » ou « haut » ou les prépositions « au-dessus de » et « en-dessous de » font référence à des positions relatives à la direction verticale, représentée par la direction V sur la figure 1b, parallèle à l'axe X.
- Les verbes « comporter », « présenter », ou « comprendre » doivent être interprétés de manière large, non limitative, sauf indications contraires.
- Sauf indications contraires, « cylindrique » fait référence à un cylindre de section circulaire.
- Un déplacement est dit « indexé » ou « discret » lorsqu'il se fait pas à pas, c'est-à-dire par des « sauts » entre deux positions d'indexation successives.
- Une pièce est dite « escamotable » lorsqu'elle peut s'effacer dans une autre pièce, de manière à ne plus faire saillie de la surface de cette autre pièce.
- Une butée « vers le bas » est un organe limitant l'insertion de l'actionneur dans le logement de localisateur.
- Une butée « vers le haut » est un organe limitant l'extraction de l'actionneur hors du logement de localisateur, et, en particulier son extraction hors du rehausseur.
- Sauf indications contraires, un trou peut être traversant ou non traversant.
- Une première pièce est conformée pour guider une deuxième pièce lorsqu'elle peut imposer un déplacement de cette deuxième pièce. Les solutions techniques pour assurer un guidage en translation et/ou en rotation d'une pièce par rapport à une autre sont bien connues de l'homme du métier. L'invention n'est pas limitée à une solution technique particulière.
- « Une rotation à partir d'une profondeur d'insertion » de l'actionneur est une rotation qui débute dans une position de l'actionneur inséré à ladite profondeur d'insertion, indépendamment de la position angulaire de l'actionneur.
- Un axe d'orientation du localisateur est un axe du localisateur qui peut être utilisé pour définir la position angulaire du localisateur par rapport à la valve. De même, un axe d'orientation de la valve est un axe de la valve qui peut être utilisé pour définir la position angulaire de la valve par rapport au localisateur.
- La « pression d'ouverture » correspond, dans une position du rotor de la valve, à la pression minimale nécessaire pour déplacer l'obturateur à l'encontre de la contrainte exercée par l'organe de rappel élastique sur l'obturateur.
- Par « ressort », on entend tout moyen élastique, et en particulier un ressort hélicoïdal, un ressort à lame, ou un bloc de matériau déformable élastiquement.
- Le terme « micro-aimant » est utilisé à des fins de clarté, pour distinguer les aimants du rotor de ceux de l'actionneur. Les aimants du rotor sont en effet beaucoup plus petits que ceux de l'actionneur, comme représenté sur les figures. « Micro » n'est donc pas limitatif.

### Brève description des figures

D'autres caractéristiques et avantages de l'invention apparaîtront encore à la lecture de la description détaillée qui va suivre et à l'examen du dessin détaillé dans lequel
- les figures 1 à 6 représentent un dispositif selon l'invention dans des configurations adoptées successivement lors de la mise en œuvre d'un procédé selon l'invention,
- la figure 7 représente, en perspective, un localisateur dans un deuxième mode de réalisation de l'invention, et
- les figures 8 à 9 représentent la clé magnétique du dispositif selon l'invention des figures 1 à 6 dans des positions basse et haute de l'actionneur, respectivement.

Chaque figure regroupe
- une représentation en perspective (indice « a »),
- une représentation en coupe selon le plan de coupe représenté sur la représentation « c » (indice « b »), et
- une représentation vue de dessus (indice « c »).

Sur les différentes figures, des références identiques ont été utilisées pour désigner des organes identiques ou analogues.

### Description détaillée

### Valve

La valve 2 représentée est une valve du type de celles décrites dans EP 688 575 ou dans EP 3 046 614.

Sur les figures, dans un souci de clarté, seuls le corps 4, le rotor 5 et les deux micro-aimants 6 du rotor 5 ont été représentés. EP 688 575 et EP 3 046 614 décrivent dans le détail le fonctionnement de telles valves.

### Dispositif

Le dispositif de réglage selon l'invention représenté comporte un localisateur 12, optionnellement un instrument de lecture, classiquement une boussole (non représentée), et une clé 16 ou « instrument de réglage ».

### Localisateur

Le localisateur 12, parfois appelé « sélecteur » ou « instrument de localisation », présente la forme générale d'une coupelle d'axe X. Il comporte un fond 22, s'étendant perpendiculairement à l'axe X et définissant des surfaces supérieures 22ₛ et inférieures 22ᵢ sensiblement plates. Une fenêtre 26 est découpée sensiblement au centre du fond 22.

La fenêtre 26 présente la forme générale de la valve 2 vue de dessus et, de préférence, des accessoires éventuellement connectés à la valve, en particulier un réservoir en amont et un dispositif anti-siphon en aval. Les dimensions de la fenêtre sont adaptées de manière à pouvoir repérer par palpation, à travers la fenêtre, la valve et lesdits accessoires, ce qui permet de positionner le localisateur dans une position précise par rapport à la valve, même sans apercevoir ladite valve. En particulier, les axes définissant la longueur de la fenêtre 26 et de la valve 2 (axe rejoignant l'entrée et la sortie de la valve 2), constituent des axes d'orientation O_{L} et O_{V}, respectivement, qui peuvent ainsi être facilement orientés parallèlement l'un à l'autre comme sur les figures 1 à 6. La fenêtre 26 est ainsi un trou de localisation.

Dans un mode de réalisation, des reliefs 28 font saillie depuis la surface inférieure 22ᵢ de manière à améliorer le positionnement du localisateur 12 sur la valve. De préférence les reliefs dessinent l'empreinte de la valve, ce qui améliore l'immobilisation du localisateur par rapport à la valve. De préférence cependant, la surface inférieure 22ᵢ est plate, ce qui minimise la distance entre le fond du localisateur et la valve, et donc facilite le réglage de la valve.

Le fond 22 est ceinturé par une paroi latérale 32 cylindrique d'axe X, qui s'étend à partir du fond 22, vers le haut, à la périphérie du fond 22. Comme détaillé ci-après, la paroi latérale 32 définit un logement de localisateur 34 configuré pour guider l'insertion de la clé 16 selon l'axe X et la rotation autour de l'axe X de ladite clé.

La paroi latérale 32 définit une surface radialement intérieure 32ᵣᵢ et une surface radialement extérieure 32ᵣₑ. Des rainures de guidage 38, s'étendant parallèlement à l'axe X et régulièrement réparties autour de l'axe X, sont ménagées sur la surface radialement intérieure 32ᵣᵢ, par exemple tous les 15 degrés. Ces rainures sont configurées pour coopérer avec deux billes escamotables élastiquement, alignées selon un diamètre, faisant saillie à la surface latérale de la clé. Les billes s'escamotent lors de la rotation de la clé entre deux positions consécutives dans lesquelles lesdites billes pénètrent dans des rainures de guidage 38. L'écart angulaire entre deux positions angulaires consécutives est par exemple de 15°. Lors de la rotation de la clé, l'opérateur est averti, par l'émission d'un clic audible et/ou par un ressenti tactile provoqués par l'enclenchement des billes dans les rainures de guidage, à chaque fois qu'une desdites positions angulaires est atteinte.

La face supérieure de la paroi latérale 32 comporte une zone de lecture 40 portant des marques de position 42, régulièrement réparties, autour de l'axe X, de préférence sur 360 degrés autour de l'axe X. L'écart angulaire entre deux marques de position 42 correspond de préférence à l'écart angulaire entre deux rainures de guidage 38. Les marques de position 42 représentent de préférence des valeurs de pression d'ouverture. En l'occurrence, ces marques de position montrent que la pression d'ouverture de la valve peut être réglée à une valeur comprise entre 20 et 420 mmH₂O.

Un repère d'alignement 43 ou « flèche d'orientation », en l'occurrence superposé à la marque « 140 mmH₂O », indique la direction de la fenêtre 26, ce qui permet d'orienter le localisateur dans le sens d'écoulement du liquide.

Dans le mode de réalisation représenté, une bague tournante 44 est montée mobile en rotation autour de l'axe X sur la paroi latérale 32. La bague tournante 44 porte un repère de position 45 qui peut être placé par l'opérateur au regard d'une des marques de position 42. Sur la figure 1a, le repère de position 45 a ainsi été placé en regard de la marque de position « 70 ». De préférence, le déplacement de la bague tournante 44 est indexé, de préférence de manière que le repère de position 45 ne puisse qu'être positionné face à des marques de position 42. Le mécanisme d'indentation peut être un mécanisme conventionnel, par exemple comportant une languette élastique dont la tête, en appui élastique sur la paroi latérale 32, pénètre dans des logements correspondants, régulièrement espacés, ménagés sur la paroi latérale 32.

La bague tournante 44 est de préférence crantée afin de faciliter sa mise en rotation par l'opérateur.

### Instrument de lecture

L'instrument de lecture peut être en particulier une boussole, un compas magnétique tel que décrit dans EP 2 192 944 ou EP 2 218 952, ou encore un compas électronique tel que décrit dans EP 231 010.

### Clé

La clé 16 comporte un actionneur 50 et un rehausseur 52 d'axe Y (confondu avec l'axe X sur les figures). Un et de préférence plusieurs ressorts hélicoïdaux 54 sont interposés entre le rehausseur 52 et l'actionneur 50 de manière à s'opposer à leur rapprochement selon l'axe Y.

L'actionneur 50 comporte un support 56 et un barreau magnétique 58 d'axe δ₅₈ portant un ensemble d'aimants d'actionneur, et solidarisée au support 56 au moyen d'une bride 59 fixée sur le support 56. Le support 56 présente la forme générale d'un plateau 60 discoïdal, d'axe Y.

Une poignée 62 fait saillie de la surface supérieure 60ₛ du plateau 60, en s'étendant radialement par rapport à l'axe Y. La poignée 62 porte un indicateur d'orientation magnétique 64 indiquant l'orientation magnétique du barreau magnétique 58. Quand la clé est insérée dans le logement de localisateur 34 et est couplée magnétiquement avec les micro-aimants 6 du rotor 5, de manière à l'entraîner en rotation, l'indicateur d'orientation magnétique 64 pointe vers la marque de position 42 donnant la pression d'ouverture de la valve.

La surface inférieure 60ᵢ du plateau 60, généralement plane, définit un logement 66 recevant le barreau magnétique 58, et des reliefs 67 facilitant la fixation de la bride 59 sur le support 56. L'axe δ₅₈ et l'indicateur d'orientation magnétique 64 sont de préférence coplanaires avec l'axe Y. L'observation de l'indicateur d'orientation magnétique 64 permet ainsi de déterminer l'orientation du barreau magnétique 58 autour de l'axe Y.

Les aimants 72 du barreau magnétique 58 sont agencés de manière à produire un champ magnétique capable de déplacer les micro-aimants de rotor de la valve afin de déverrouiller le rotor, puis de l'entraîner en rotation. L'agencement des aimants 72 doit donc être déterminé en fonction de la position et de l'agencement des micro-aimants 6 du rotor de la valve.

L'adaptation du barreau magnétique 58 en fonction de la disposition des micro-aimants 6 à cet effet ne pose pas de difficultés particulières.

De préférence, pour une efficacité optimale, les aimants 72 sont agencés de manière à produire un champ magnétique qui est maximal dans le plan passant par l'axe Y et contenant l'axe δ₅₈, du barreau magnétique ou « plan de champ maximal ».

De préférence, les aimants 72 sont agencés de manière que le champ magnétique produit soit symétrique par rapport au plan de champ maximal et par rapport à un plan perpendiculaire au plan de champ maximal et passant par l'axe Y.

De préférence, l'ensemble des aimants 72 comporte au moins 1, au moins 2, de préférence 3 aimants dont l'axe Nord-Sud est radial par rapport à l'axe Y, dit « aimants radiaux 72ᵣ ».

De préférence, l'ensemble des aimants 72 comporte au moins 1, au moins 2, au moins 3, de préférence 4 aimants dont l'axe Nord-Sud est parallèle à l'axe Y, ou « aimants longitudinaux » 72ₗ.

De préférence, chaque aimant radial 72ᵣ est pris en sandwich entre deux aimants longitudinaux 72ₗ. De préférence encore, le long de l'axe δ₅₈ du barreau magnétique, les aimants se succèdent de la manière suivante : 72ₗ, 72ᵣ, 72ₗ, 72ᵣ (aimant centré sur l'axe Y), 72ₗ, 72ᵣ et 72ₗ.

De préférence encore, dès que deux aimants 72ₗ prennent en sandwich un aimant radial 72ᵣ, leur orientation magnétique est inversée. Autrement dit, le premier de ces aimants longitudinaux présente un pôle Nord au-dessus du pôle Sud alors que l'autre de ces aimants présente un pôle Sud au-dessus du pôle Nord.

De préférence encore, dès que deux aimants radiaux 72ᵣ prennent en sandwich un aimant longitudinal 72ₗ, leur orientation magnétique est inversée. Autrement dit, les pôles de ces aimants radiaux qui se font face (séparés par ledit aimant longitudinal) sont de même nature, par exemple sont deux pôles Nord ou deux pôles Sud.

De préférence, tous les aimants 72 ont une longueur, mesurée selon l'axe Y, qui est identique.

De préférence, la largeur d'un aimant 72, mesurée selon l'axe δ₅₈, est d'autant plus petite que l'aimant 72 considéré est proche de l'axe Y. De préférence, les aimants 72 sont agencés suivant une configuration de Halbach. L'inventeur a découvert que cette configuration est particulièrement efficace.

De préférence, l'actionneur comporte une semelle 74, de préférence sensiblement cubique-rectangle, sur la face inférieure de laquelle les aimants 72 sont fixés. La semelle 74 est de préférence métallique et s'étend de préférence dans un plan perpendiculaire à l'axe Y, de préférence selon l'axe δ₅₈ du barreau magnétique.

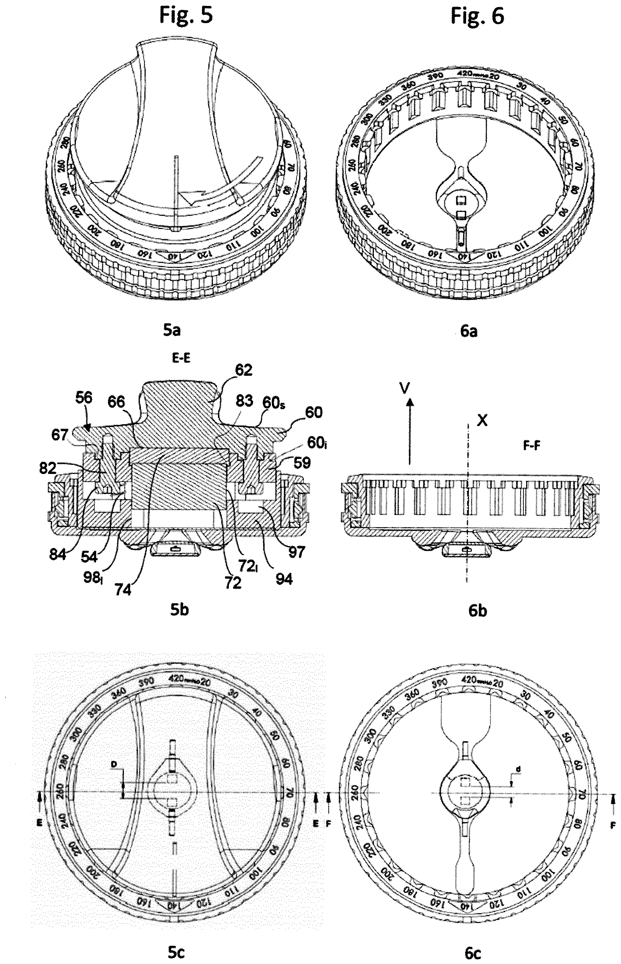

Tout déplacement de la semelle 74 dans un plan perpendiculaire à l'axe Y est entravé par la complémentarité de forme entre la semelle 74 et le logement 66 du support 56 (voir les figures 2b et 5b).

Tout déplacement de la semelle 74 parallèlement à l'axe Y est entravé par le serrage de la semelle 74 contre le fond du logement 66, au moyen de vis de fixation 82.

Plus précisément, la semelle 74 dépasse latéralement des aimants 72 de manière à former deux épaulements 83. La bride 59 est percée par un orifice à travers lequel les aimants 72 peuvent faire saillie de la surface inférieure de la bride 59. Des cornières sont cependant ménagées dans la surface définissant ledit orifice de manière que lesdites cornières puissent prendre appui sur lesdits épaulements 83. Chaque vis de fixation 82 de la bride 59 sur le support 56 traverse un orifice 85 de la bride 59 et est vissée dans un alésage respectif du support. Chaque vis de fixation 82 comporte une tête 84 qui prend appui de la surface inférieure, sensiblement plate, de la bride 59, de manière à presser la semelle 74 dans le logement 66.

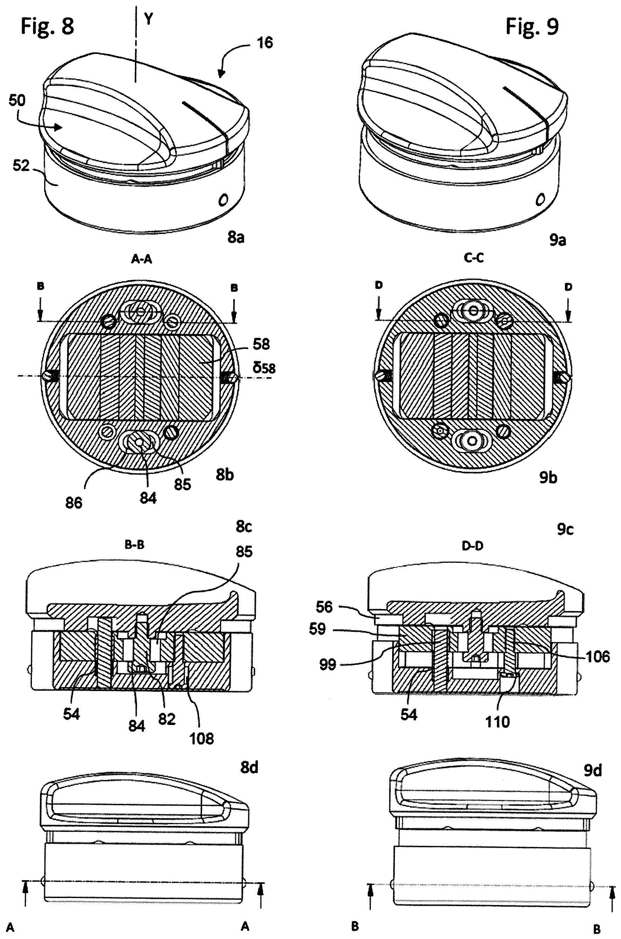

Dans un mode de réalisation préféré, la fixation de la bride 59 sur le support 56 autorise cependant une translation du support, et donc du barreau magnétique 58 rigidement fixé sur le support 56, par rapport à la bride 59, de préférence suivant l'axe δ₅₈ du barreau magnétique. A cet effet, l'orifice 85 est de préférence de section oblongue, le grand axe de ladite section étant parallèle à la direction de la translation (figures 8b et 9b). Comme représenté sur les figures 8b et 9b, la tête 84 ne prend alors appui que sur les bords latéraux 86 de l'ouverture par laquelle l'orifice 85 débouche sur la surface inférieure de la bride 59.

### Rehausseur

Le rehausseur 52 est destiné à guider la translation de l'actionneur 50 le long de l'axe Y.

Il comporte un fond de rehausseur 92 présentant la forme d'un disque plein d'axe Y, et une base 94 qui, vers le haut, se termine par une jupe 96. La base 94 est percée de manière à définir, avec le fond 92, une cavité 98 apte à recevoir l'ensemble d'aimants 72 lors de l'enfoncement de l'actionneur selon l'axe Y.

La surface supérieure de la base 94 définit encore des orifices 97 de réception des têtes 84 des vis de fixation de la bride sur le support.

La surface radialement intérieure 96ᵣᵢ de la jupe 96 et/ou la surface latérale 98ₗ de la cavité 98 guide(nt) l'enfoncement de l'actionneur, selon l'axe Y, vers le fond de rehausseur 92, par coopération avec la surface radialement extérieure 59ᵣₑ de la bride 59 et la surface latérale 72ₗ de l'ensemble d'aimants 72, respectivement.

De préférence, l'actionneur n'est mobile qu'en translation, selon l'axe Y, par rapport au rehausseur, de préférence par coopération des surfaces latérales 98ₗ et 72ₗ.

Le nombre de ressorts 54 n'est pas limité. De préférence, il est supérieur à 2, supérieur à 3, voire supérieur à 4, et/ou inférieur à 10. Les ressorts sont de préférence répartis équi-angulairement autour de l'axe Y. De préférence encore, ils s'étendent sensiblement parallèlement à l'axe Y, ce qui optimise leur efficacité.

De préférence encore, chaque ressort 54 est enfilé sur une tige de guidage 99 fixée sur le rehausseur ou sur l'actionneur et qui, lorsque l'actionneur est enfoncé dans le rehausseur, coulisse dans un logement correspondant ménagé sur l'actionneur ou le rehausseur, respectivement, comme représenté sur les figures 8c et 9c.

De préférence, le rehausseur est maintenu solidaire de l'actionneur, par exemple par fixation de vis 106 sur la bride 59, à travers un orifice 108 ménagé dans le rehausseur, comme représenté sur les figures 8c et 9c. L'orifice 108 est de préférence conformé pour guider le coulissement de la tête 110 de la vis 106 parallèlement à l'axe Y, tout en lui servant de deuxième butée pour définir la position haute de l'actionneur, comme représenté sur la figure 9.

Les ressorts 54, hélicoïdaux, sont interposés entre l'actionneur 50 et le rehausseur 52, de manière à s'opposer à l'enfoncement de l'actionneur dans le rehausseur. Au repos, c'est-à-dire en l'absence de poussée d'insertion sur l'actionneur, les ressorts 54 maintiennent l'actionneur à la deuxième profondeur d'insertion (position haute de l'actionneur représenté sur la figure 9).

### Fonctionnement

Le fonctionnement du dispositif résulte directement de la description qui précède.

Dans la position initiale représentée sur la figure 1, les deux micro-aimants du rotor de la valve sont écartés l'un de l'autre d'une distance d, dans une position « rapprochée ». De préférence, cette position rapprochée résulte de l'attraction mutuelle des deux micro-aimants.

Dans la position rapprochée, les deux micro-aimants maintiennent de préférence des ergots de verrouillage du rotor respectifs dans des cavités de la chambre, empêchant ainsi toute rotation du rotor. Ce mode de réalisation peut par exemple correspondre aux figures 1 et 2 de EP 688 575. La position rapprochée des micro-aimants est donc une position de verrouillage du rotor de la valve.

Sur les différentes figures, la peau du patient n'a pas été représentée.

La valve peut être notamment disposée sous la peau du crâne, du cou, de la fosse claviculaire, au niveau du dos ou de la hanche.

L'aimant d'actionneur est de préférence configuré pour exercer un champ magnétique adapté pour, lorsque la valve est à l'air libre et que le localisateur est disposé perpendiculairement à l'axe de rotation du rotor de la valve, à une distance de la valve supérieure à 2 mm, supérieure à 3 mm, supérieure à 5 mm, supérieure à 8 mm, et/ou inférieure à 30 mm, inférieure à 25 mm, inférieure à 20 mm, inférieure à 15 mm, inférieure à 12 mm, ou inférieure à 10 mm, déverrouiller le rotor à la première profondeur d'insertion et être couplé en rotation avec le rotor à la deuxième profondeur d'insertion.

La valve 2 étant implantée, elle s'étend entre la valve 2 et le fond du localisateur 12. La valve n'est donc pas visible par l'opérateur, qui peut cependant la repérer par palpation.

**A l'étape a),** l'opérateur doit repérer précisément la valve 2 et positionner le localisateur en conséquence.

A cet effet, il dispose le localisateur 12 contre la peau du patient, de manière à disposer la valve dans la fenêtre 26 du localisateur. La valve 2 pénètre alors légèrement, par déformation de la peau, dans la fenêtre 26 du localisateur.

L'axe d'orientation Ov de la valve s'étend alors parallèlement à l'axe d'orientation O_{L} du localisateur. En outre, l'axe de rotation R du rotor est alors sensiblement confondu avec l'axe X du logement du localisateur.

L'axe de rotation R du rotor peut passer par le centre du corps de valve. Il peut être alternativement légèrement décentré avec le centre du corps de valve, et donc avec l'axe longitudinal Ov de la valve, notamment afin de ménager, entre le rotor et le corps de valve, un espace pour loger l'organe de rappel élastique en appui sur l'obturateur. En conséquence, l'axe X du logement du localisateur peut ne pas couper l'axe d'orientation O_{L} du localisateur (axe longitudinal de la fenêtre 26).

Le repère d'alignement 43 permet d'orienter le localisateur dans le sens d'écoulement du liquide.

Une orientation du localisateur par rapport à la valve est par exemple décrite dans EP 2 192 944.

Pour modifier la position angulaire du rotor, l'opérateur procède de la manière suivante :
**A l'étape b),** l'opérateur détermine la position angulaire du rotor de la valve autour de son axe avec l'instrument de lecture, par exemple un compas magnétique ou « boussole ».

De manière connue, comme décrit dans EP 2 192 944, la boussole est de préférence introduite dans le logement de localisateur, de manière que l'axe de rotation de l'aiguille de la boussole soit centré sur l'axe X du logement de localisateur, qui lui-même est sensiblement centré sur l'axe de rotation R du rotor de la valve. De préférence, la boussole est également utilisée pour améliorer la précision du positionnement du localisateur afin que l'axe de rotation R du rotor soit confondu avec l'axe X du logement du localisateur.

L'aiguille de la boussole s'oriente alors suivant l'axe δ₆ des deux micro-aimants 6, radial par rapport à l'axe de rotation R du rotor. Sur la figure 1a, il apparaît que le repère de position 45 est alors aligné avec la marque de position « 70 ».

De préférence, l'opérateur repère l'axe δ₆ en faisant tourner la bague tournante 44 de manière à aligner le repère de position 45 avec cet axe. L'opérateur peut ainsi mémoriser que la pression d'ouverture de la valve, dans la configuration initiale, est en l'occurrence de 70 mmH₂O.

Il extrait ensuite la boussole hors du logement de localisateur.

Les étapes suivantes sont destinées à modifier la pression d'ouverture de manière à ce qu'elle atteigne une pression d'ouverture de consigne de 140 mmH₂O, par exemple.

**A l'étape c),** l'opérateur dispose l'actionneur afin que l'indicateur d'orientation magnétique 64 soit dirigé vers le repère de position 45 « 70 » indiquant l'orientation de l'axe δ₆ des micro-aimants de la valve. L'axe δ₅₈ du barreau magnétique est alors coplanaire à l'axe δ₆ des micro-aimants et coplanaire à l'axe Y.

La clé 16 est alors insérée dans le logement de localisateur, suivant l'axe X. Cette insertion est guidée par le contact entre la surface radialement extérieure 94ᵣₑ de la base du rehausseur et la surface radialement intérieure 32ᵣᵢ de la paroi latérale 32 du localisateur. L'insertion se poursuit jusqu'à ce que le rehausseur entre en butée avec le fond 22 du localisateur. Comme représenté sur la figure 2b, le fond 92 du rehausseur repose de préférence sensiblement sur toute sa surface sur le fond 22 du localisateur.

Les ressorts 54 s'opposent alors à l'insertion de l'actionneur dans le rehausseur (figure 2).

Dans cette position de l'actionneur, dite « position haute », les aimants 72 sont trop éloignés pour écarter l'un de l'autre les micro-aimants du rotor.

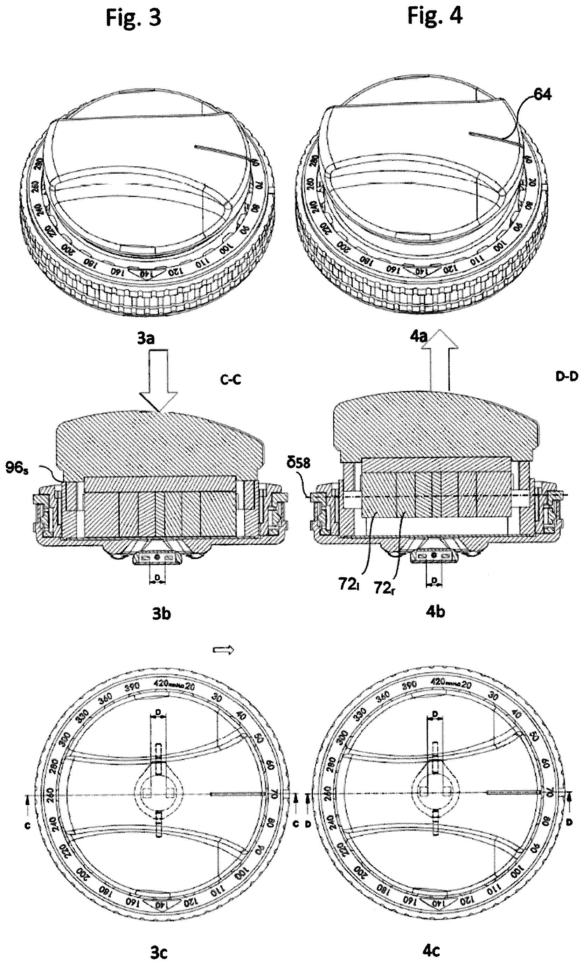

L'opérateur exerce alors une poussée d'insertion, selon l'axe Y, afin de poursuivre l'insertion de l'actionneur dans le trou d'actionneur, par coulissement sur le rehausseur, jusqu'à la première profondeur d'insertion (flèche sur la figure 3). L'actionneur est alors en « position basse ».

La première profondeur d'insertion correspond avantageusement à une insertion maximale, déterminée par l'entrée en butée de l'actionneur avec le rehausseur, par exemple par entrée en contact de l'actionneur avec le chant 96ₛ de la jupe 96, et/ou la surface supérieure 94ₛ de la base 94 du rehausseur et/ou le fond 92 du rehausseur et/ou le fond d'orifices de réception des têtes de vis de fixation de la bride, comme représenté sur la figure 3b. Le chant de la jupe 96, la surface supérieure de la base 94, le fond du rehausseur et le fond des orifices de réception sont des exemples de « première butée » déterminant la première profondeur d'insertion de l'actionneur.

En variante, le rehausseur pourrait être dépourvu de fond et les aimants 72 pourraient entrer en butée avec le fond du localisateur, qui constituerait alors une première butée déterminant la première profondeur d'insertion.

A la première profondeur d'insertion et après alignement de l'indicateur d'orientation magnétique 64 sur le repère de position 45, les aimants 72 de l'actionneur sont proches des micro-aimants du rotor et exercent un champ magnétique suffisamment puissant pour les écarter l'un de l'autre. La distance entre les deux micro-aimants augmente jusqu'à une valeur D. L'action des micro-aimants n'entrave alors plus la rotation du rotor. Les micro-aimants passent ainsi de la position rapprochée (figures 1 et 2), dans laquelle le rotor est verrouillé, à une position écartée (figures 3 à 5), dans laquelle le rotor est déverrouillé.

De préférence, avant de tourner l'actionneur, l'opérateur déplace la poignée suivant l'axe δ₅₈ du barreau magnétique. Ce déplacement entraine celui des aimants 72 de l'actionneur, ce qui agit sur les micro-aimants du rotor qui leur sont accouplés. En répétant ces déplacements, dans un sens puis dans l'autre (mouvement de va-et-vient), l'opérateur peut ainsi légèrement secouer et, le cas échéant, débloquer alternativement les micro-aimants, ce qui facilite leur déverrouillage.

La rotation de l'actionneur inséré à la première profondeur d'insertion dans le logement de localisateur permet d'entraîner en rotation le rotor de la valve. Cet entraînement n'est cependant pas toujours fiable.

**A l'étape d),** selon l'invention, l'opérateur retire partiellement l'actionneur du logement de localisateur, jusqu'à la deuxième profondeur d'insertion, définie par la position au repos des ressorts 54.

Pour retirer partiellement l'actionneur du logement de localisateur, il suffit donc à l'opérateur de relâcher la pression sur l'actionneur. Ce dernier remonte alors, sous l'effet des ressorts 54, jusqu'à la position de repos représentée sur la figure 4, identique à la position de la figure 2.

Comme le montre la figure 4b, les micro-aimants de la valve restent cependant en position écartée. Sans être limité par cette théorie, l'inventeur explique ce phénomène par la nécessité d'exercer une force plus élevée pour écarter les micro-aimants l'un de l'autre (déverrouillage) que pour les maintenir dans la position écartée.

De manière surprenante, l'inventeur a également constaté que, dans la position de l'actionneur correspondant à la deuxième profondeur d'insertion, représentée sur la figure 4b, l'entraînement en rotation du rotor déverrouillé était beaucoup plus fiable que dans la position de l'actionneur correspondant à la première profondeur d'insertion, représentée sur la figure 3.

Dans l'exemple représenté, la deuxième profondeur d'insertion de l'actionneur est donc déterminée par le rehausseur, qui constitue ainsi une « deuxième butée d'insertion ».

L'ensemble constitué par le rehausseur et les ressorts au repos forme également un guide de la rotation de l'actionneur 50 autour de l'axe X, dans un plan perpendiculaire à l'axe X. Quelle que soit la position angulaire de l'actionneur, les ressorts s'opposent en effet à une poursuite de l'insertion de l'actionneur.

**A l'étape e),** l'opérateur peut tourner la clé jusqu'à une position correspondant à la pression d'ouverture de consigne, représentée sur la figure 5 et correspondant, par exemple, à 140 mmH₂O. Les ressorts 54 permettent avantageusement à l'opérateur de maintenir la profondeur d'insertion de l'actionneur à la deuxième profondeur d'insertion. La fiabilité en est améliorée.

Un ensemble d'aimants 72 agencé selon une configuration de Halbach permet avantageusement un déverrouillage du rotor et un entraînement en rotation du rotor particulièrement fiables.

**A l'étape f),** l'opérateur écarte l'actionneur de la valve. Les forces de rapprochement des micro-aimants redeviennent alors supérieures aux forces d'écartement générées par l'actionneur, ce qui conduit à les rapprocher, jusqu'à la distance d, dans laquelle elles verrouillent en rotation le rotor, comme représenté sur la figure 6.

Dans un mode de réalisation, la rotation de la clé magnétique est indexée par rapport au localisateur, de manière que l'indicateur d'orientation magnétique 64 ne puisse être positionné que face à une marque de position 42. De préférence, l'indentation est déterminée pour que, lorsque l'action de l'actionneur sur les micro-aimants devient faible et que les micro-aimants ont tendance à se rapprocher, les ergots soient sensiblement en face d'une cavité du corps de la valve et qu'ils puissent pénétrer dans ces cavités sous l'effet du rapprochement mutuel des micro-aimants.

Pour écarter l'actionneur de la valve, l'actionneur peut être extrait du localisateur.

Comme cela apparaît clairement à présent, l'invention fournit une solution simple et fiable pour régler la pression d'ouverture d'une valve dont le rotor est verrouillable au moyen de micro-aimants. Bien entendu, l'invention n'est cependant pas limitée aux modes de réalisation décrits et représentés, fournis à des fins illustratives seulement.

En particulier, d'autres moyens que le rehausseur pourraient être utilisés pour déterminer les première et deuxième profondeurs d'insertion.

Le rehausseur n'est pas nécessairement solidaire de l'actionneur.

Le rehausseur pourrait être intégré dans le localisateur.

Dans un mode de réalisation, la rotation de l'actionneur n'entraîne pas celle du rehausseur.

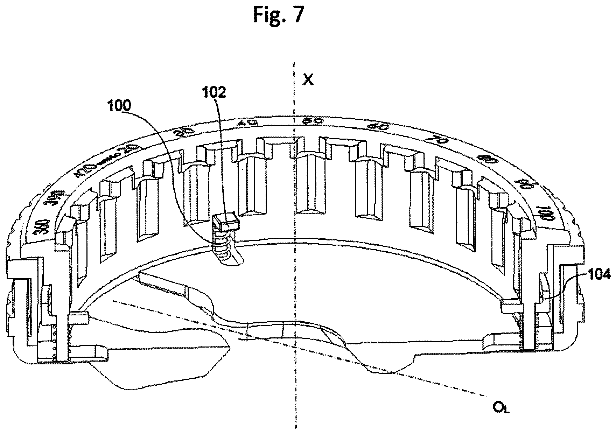

Comme représenté sur la figure 7, des ressorts 100 équivalents aux ressorts 54 pourraient prendre appui directement sur le localisateur, par exemple sur le fond du localisateur, le rehausseur devenant optionnel.

Dans le mode de réalisation représenté sur la figure 7, des ressorts sont par exemple logés dans l'épaisseur de la paroi latérale du localisateur. Chaque ressort porte une patte 102, en appui sur l'extrémité du ressort opposée à l'extrémité en appui sur le localisateur, qui fait saillie à l'intérieur du logement de localisateur. Les pattes s'opposent ainsi, sous l'effet des ressorts, à l'insertion de l'actionneur au-delà de la deuxième profondeur d'insertion.

De préférence, les pattes peuvent s'effacer dans le fond du localisateur, ce qui permet à la première profondeur d'insertion, aux aimants d'être les plus proches possible de la valve.

Dans le mode de réalisation représenté, les pattes sont reliées les unes aux autres par un anneau circulaire 104, qui est mobile dans un espace annulaire, d'axe X, ménagé dans la paroi latérale du localisateur.

Dans un mode de réalisation, la première profondeur d'insertion et/ou la deuxième profondeur d'insertion sont définies par des pions, par exemple sous la forme de billes, faisant élastiquement saillie de la paroi latérale du localisateur ou de l'actionneur sous l'effet de ressorts s'étendant de préférence radialement par rapport à l'axe X. En pénétrant dans des logements correspondant de l'actionneur ou du localisateur, respectivement, les pions peuvent ainsi s'opposer à un déplacement de l'actionneur, selon l'axe X, hors de la première profondeur d'insertion et/ou la deuxième profondeur d'insertion. De préférence, les pions peuvent cependant s'effacer dans la paroi latérale du localisateur ou de l'actionneur sous l'effet d'une poussée d'insertion et/ou d'une traction d'extraction exercées par l'opérateur sur l'actionneur.

Les caractéristiques des différents modes de réalisation, en particulier des modes de réalisation représentés, peuvent être combinées, sauf incompatibilité technique.

### Référence des figures

2 : Valve
4 : Corps de valve
5 : Rotor de valve
6 : Micro-aimants du rotor
12 : Localisateur
16 : Clé magnétique
22 : Fond du localisateur
26 : Fenêtre dans le fond du localisateur
28 : Reliefs
32 : Paroi latérale du localisateur
34 : Logement de localisateur
38 : Rainure de guidage
40 : Zone de lecture
42 : Marques de position portées par la zone de lecture 40
43 : Repère d'alignement
44 : Bague tournante du localisateur
45 : Repère de position porté par la bague tournante 44
50 : Actionneur
52 : Rehausseur
54 : Ressorts interposés entre le rehausseur et l'actionneur
56 : Support
58 : Barreau magnétique de l'actionneur
59 : Bride de fixation du barreau magnétique 58 sur le support 56
60 : Plateau de l'actionneur
62 : Poignée de l'actionneur
64 : Indicateur d'orientation magnétique
66 : Logement du plateau 60 recevant le barreau magnétique 58
67 : Relief de fixation de la bride sur le support
72 : Aimants de l'actionneur
74 : Semelle
83 : Epaulement
82 : Vis de fixation de la bride sur le support
84 : Têtes de vis de fixation de la bride sur le support
85 : Orifice de fixation de la bride sur le support
86 : Bord latéral de l'ouverture par laquelle l'orifice 85 débouche sur la surface inférieure de la bride 59
92 : Fond de rehausseur
94 : Base du rehausseur
96 : Jupe du rehausseur
97 : Orifices de réception des têtes des vis
98 : Cavité ménagée dans la base 94
99 : Tige de guidage de ressort
100 : Ressorts du localisateur
102 : Pattes poussées par les ressorts du localisateur
104 : Anneau circulaire reliant les pattes
106 : Vis de fixation du rehausseur sur la bride
108 : Orifice de fixation du rehausseur sur la bride
110 : Tête de vis 106
X : Axe du logement du localisateur
Y : Axe de la clé magnétique
R : Axe de rotation du rotor de la valve
Ov : Axe longitudinal de la valve
O_{L} : Axe longitudinal de la fenêtre du localisateur
δ₆ : Axe des micro-aimants du rotor
δ₅₈ : Axe du barreau magnétique

## Revendications

1. Dispositif de traitement comportant
- une valve implantable comportant :
- un corps (4) définissant une chambre interne,
- un conduit d'entrée et un conduit d'évacuation ménagés dans la paroi latérale de ladite chambre interne et aptes à être respectivement connectés à un cathéter d'amenée et un cathéter de drainage de liquide,
- un rotor (5) apte à tourner dans ladite chambre interne autour d'un axe de rotation (R), le rotor comportant au moins un micro-aimant (6) mobile entre des positions de verrouillage et de déverrouillage du rotor, dans lesquelles il interdit et autorise la rotation dudit rotor, respectivement, une rotation du rotor modifiant la pression d'ouverture de la valve,
- un obturateur,
- un organe de rappel élastique comprimant l'obturateur dans son siège de manière à réguler, et le cas échéant bloquer le passage de liquide dans la chambre interne,
- un dispositif de réglage de la pression d'ouverture de ladite valve,
le dispositif de traitement étant **caractérisé en ce que** le dispositif de réglage comporte :
- un localisateur (12) de valve définissant un logement de localisateur (34) d'axe X;
- une clé (16) comportant un actionneur (50) comportant un aimant (72) d'actionneur, l'actionneur étant insérable, selon l'axe X, dans le logement de localisateur, à une première profondeur d'insertion et à une deuxième profondeur d'insertion inférieure à la première profondeur d'insertion, le dispositif de réglage comportant un guide (52,54) de la rotation de l'actionneur autour de l'axe X depuis au moins une position angulaire de l'actionneur autour de l'axe X dans laquelle l'actionneur est à la deuxième profondeur d'insertion, l'aimant d'actionneur exerçant un champs magnétique adapté pour, lorsque le localisateur est disposé contre la peau d'un patient sous laquelle la valve a été implantée, la valve étant disposée dans une fenêtre (26) du localisateur, déverrouiller le rotor à la première profondeur d'insertion et être couplé en rotation avec le rotor à la deuxième profondeur d'insertion.

2. Dispositif de traitement selon la revendication immédiatement précédente, dans lequel le guide est conformé pour guider la rotation de l'actionneur autour de l'axe X depuis n'importe quelle position angulaire de l'actionneur autour de l'axe X dans laquelle l'actionneur est à la deuxième profondeur d'insertion, dans un plan de rotation sensiblement perpendiculaire à l'axe X.

3. Dispositif de traitement selon l'une quelconque des deux revendications immédiatement précédentes, dans lequel la différence entre les première et deuxième profondeurs d'insertion, mesurées selon l'axe X, est supérieure à 5 millimètres.

4. Dispositif de traitement selon l'une quelconque des trois revendications immédiatement précédentes, comportant des première (96ₛ ; 94ₛ ; 92) et deuxième (52,54) butées définissant les première et deuxième profondeurs d'insertion, respectivement.

5. Dispositif de traitement selon l'une quelconque des quatre revendications immédiatement précédentes, dans lequel la clé comporte un rehausseur (52) sur lequel l'actionneur est monté mobile en translation selon un axe Y de la clé qui, lorsque la clé est insérée dans le logement de localisateur, se confond avec l'axe X du logement de localisateur.

6. Dispositif de traitement selon la revendication immédiatement précédente, comportant un ressort (54) interposé entre l'actionneur et le rehausseur ou un ressort (100) interposé entre l'actionneur et le localisateur, de manière à s'opposer à une insertion de l'actionneur, selon l'axe Y, au-delà de la deuxième profondeur d'insertion.

7. Dispositif de traitement selon la revendication immédiatement précédente, dans lequel ledit un ressort s'étend selon un axe parallèle à l'axe X et/ou à l'axe Y ou dans un plan perpendiculaire à l'axe X ou à l'axe Y.

8. Dispositif de traitement selon l'une quelconque des deux revendications immédiatement précédentes, dans lequel ledit ressort est logé dans un logement et, du côté de l'ouverture du logement, et configuré de manière à pousser un pion vers l'axe X ou vers l'axe Y.

9. Dispositif de traitement selon l'une quelconque des quatre revendications immédiatement précédentes, dans lequel l'actionneur est guidé en translation sur le rehausseur, selon l'axe Y.

10. Dispositif de traitement selon l'une quelconque des neuf revendications immédiatement précédentes, dans lequel l'actionneur comporte un ensemble d'aimants (72) agencés suivant une configuration de Halbach.

11. Dispositif de traitement selon l'une quelconque des six revendications immédiatement précédentes, dans lequel l'actionneur comporte un support (56) sur lequel ledit aimant (72) d'actionneur est rigidement fixé, une bride (59) montée mobile en translation, selon l'axe Y, par rapport au rehausseur (52) et des moyens (82;85) pour empêcher tout déplacement du support selon l'axe Y, par rapport à la bride, tout en autorisant un déplacement du support perpendiculairement à l'axe Y, par rapport à la bride.

12. Dispositif de traitement selon l'une quelconque des revendications précédentes, dans lequel l'aimant d'actionneur est configuré pour exercer un champ magnétique adapté pour, lorsque la valve est à l'air libre et que le localisateur est disposé perpendiculairement à l'axe de rotation du rotor de la valve,
- ne déverrouiller le rotor que lorsque l'aimant d'actionneur est à une distance du rotor inférieure à 20 mm ; et/ou
- n'être couplé en rotation avec le rotor que lorsque l'aimant d'actionneur est à une distance du rotor inférieure à 30 mm.

## Patentansprüche

1. Behandlungsvorrichtung, welche umfasst:
- ein implantierbares Ventil, welches umfasst:
- einen Körper (4), der eine innere Kammer definiert,
- einen Eintrittskanal und einen Ablasskanal, die in der Seitenwand der inneren Kammer ausgebildet sind und mit einem Zuführungskatheter bzw. einem Katheter zur Drainage von Flüssigkeit verbindbar sind,
- einen Rotor (5), der in der Lage ist, in der inneren Kammer um eine Drehachse (R) zu rotieren, wobei der Rotor wenigstens einen Mikromagneten (6) umfasst, der zwischen Positionen der Verriegelung und der Entriegelung des Rotors beweglich ist, in welchen er die Rotation des Rotors verhindert bzw. ermöglicht, wobei eine Rotation des Rotors den Öffnungsdruck des Ventils ändert,
- ein Verschlussstück,
- ein elastisches Rückstellorgan, welches das Verschlussstück in seinem Sitz zusammendrückt, um so den Durchfluss von Flüssigkeit in der inneren Kammer einzustellen und gegebenenfalls zu blockieren,
- eine Vorrichtung zum Einstellen des Öffnungsdrucks des Ventils,
wobei die Behandlungsvorrichtung **dadurch gekennzeichnet ist, dass** die Vorrichtung zum Einstellen umfasst:
- einen Ventillokalisator (12), der einen Lokalisatoraufnahmeraum (34) mit einer Achse X definiert;
- einen Schalter (16), der einen Aktuator (50) umfasst, der einen Aktuatormagneten (72) umfasst, wobei der Aktuator entlang der Achse X in den Lokalisatoraufnahmeraum mit einer ersten Einsetztiefe und mit einer zweiten Einsetztiefe, die kleiner als die erste Einsetztiefe ist, einsetzbar ist, wobei die Vorrichtung zum Einstellen eine Führung (52, 54) der Drehung des Aktuators um die Achse X von wenigstens einer Winkelposition des Aktuators um die Achse X aus, in welcher sich der Aktuator in der zweiten Einsetztiefe befindet, umfasst,
wobei der Aktuatormagnet ein Magnetfeld erzeugt, das dafür eingerichtet ist, wenn der Lokalisator an der Haut eines Patienten anliegend angeordnet ist, unter welcher das Ventil implantiert worden ist, wobei das Ventil in einem Fenster (26) des Lokalisators angeordnet ist, in der ersten Einsetztiefe den Rotor zu entriegeln und in der zweiten Einsetztiefe mit dem Rotor drehgekoppelt zu werden.

2. Behandlungsvorrichtung nach dem unmittelbar vorhergehenden Anspruch, wobei die Führung dafür ausgebildet ist, die Drehung des Aktuators um die Achse X von einer beliebigen Winkelposition des Aktuators um die Achse X aus, in welcher sich der Aktuator in der zweiten Einsetztiefe befindet, in einer Rotationsebene zu führen, die im Wesentlichen senkrecht zur Achse X ist.

3. Behandlungsvorrichtung nach einem der zwei unmittelbar vorhergehenden Ansprüche, wobei die Differenz zwischen der ersten und der zweiten Einsetztiefe, entlang der Achse X gemessen, größer als 5 Millimeter ist.

4. Behandlungsvorrichtung nach einem der drei unmittelbar vorhergehenden Ansprüche, welche einen ersten (96ₛ; 94ₛ; 92) und einen zweiten (52, 54) Anschlag umfasst, welche die erste bzw. zweite Einsetztiefe definieren.

5. Behandlungsvorrichtung nach einem der vier unmittelbar vorhergehenden Ansprüche, wobei der Schalter einen Sockel (52) umfasst, auf welchem der Aktuator translatorisch bewegbar entlang einer Achse Y des Schalters angebracht ist, welche, wenn der Schalter in den Lokalisatoraufnahmeraum eingesetzt ist, mit der Achse X des Lokalisatoraufnahmeraums zusammenfällt.

6. Behandlungsvorrichtung nach dem unmittelbar vorhergehenden Anspruch, welche eine Feder (54), die zwischen dem Aktuator und dem Sockel angeordnet ist, oder eine Feder (100), die zwischen dem Aktuator und dem Lokalisator angeordnet ist, umfasst, derart, dass sie einem Einsetzen des Aktuators entlang der Achse Y über die zweite Einsetztiefe hinaus entgegenwirkt.

7. Behandlungsvorrichtung nach dem unmittelbar vorhergehenden Anspruch, wobei sich die eine Feder entlang einer Achse erstreckt, die zur Achse X und/oder zur Achse Y parallel ist, oder in einer Ebene, die zur Achse X oder zur Achse Y senkrecht ist.

8. Behandlungsvorrichtung nach einem der zwei unmittelbar vorhergehenden Ansprüche, wobei die Feder in einem Aufnahmeraum aufgenommen ist, und auf der Seite der Öffnung des Aufnahmeraums, und so ausgebildet ist, dass sie einen Zapfen zur Achse X hin oder zur Achse Y hin drückt.

9. Behandlungsvorrichtung nach einem der vier unmittelbar vorhergehenden Ansprüche, wobei der Aktuator auf dem Sockel entlang der Achse Y translatorisch geführt wird.

10. Behandlungsvorrichtung nach einem der neun unmittelbar vorhergehenden Ansprüche, wobei der Aktuator eine Anordnung von Magneten (72) umfasst, die gemäß einem Halbach-Array angeordnet sind.

11. Behandlungsvorrichtung nach einem der sechs unmittelbar vorhergehenden Ansprüche, wobei der Aktuator einen Halter (56), auf welchem der Aktuatormagnet (72) starr befestigt ist, einen Flansch (59), der translatorisch verschiebbar entlang der Achse Y in Bezug auf den Sockel (52) angebracht ist, und Mittel (82; 85) zum Verhindern jeder Verlagerung des Halters entlang der Achse Y in Bezug auf den Flansch bei gleichzeitigem Ermöglichen einer Verlagerung des Halters senkrecht zur Achse Y in Bezug auf den Flansch umfasst.

12. Behandlungsvorrichtung nach einem der vorhergehenden Ansprüche, wobei der Aktuatormagnet dafür ausgelegt ist, ein Magnetfeld zu erzeugen, das dafür eingerichtet ist, wenn das Ventil freiliegt und wenn der Lokalisator senkrecht zur Drehachse des Rotors des Ventils angeordnet ist,
- den Rotor nur zu entriegeln, wenn sich der Aktuatormagnet in einem Abstand vom Rotor befindet, der kleiner als 20 mm ist; und/oder
- nur mit dem Rotor drehgekoppelt zu werden, wenn sich der Aktuatormagnet in einem Abstand vom Rotor befindet, der kleiner als 30 mm ist.

## Claims

1. Treatment device having
- an implantable valve having:
- a body (4) defining an internal chamber,
- an admission conduit and a discharge conduit that are formed in the side wall of said internal chamber and are able to be connected, respectively, to a liquid delivery catheter and a liquid drainage catheter,
- a rotor (5) able to turn in said internal chamber about an axis of rotation (R), the rotor having at least one micro-magnet (6) movable between positions of locking and unlocking of the rotor, in which positions it respectively prohibits and permits the rotation of said rotor, a rotation of the rotor modifying the opening pressure of the valve,
- a shutter,
- an elastic return member compressing the shutter in its seat in such a way as to regulate and, if appropriate, block the passage of liquid into the internal chamber,
- a device for adjusting the opening pressure of said valve,
the treatment device being **characterized in that** the adjustment device has:
- a valve locator (12) defining a locator receptacle (34) of axis X;
- a key (16) having an actuator (50) with an actuator magnet (72), the actuator being insertable, along the axis X, into the locator receptacle to a first insertion depth and to a second insertion depth less than the first insertion depth, the adjustment device having a guide (52, 54) for guiding the rotation of the actuator about the axis X from at least one angular position of the actuator about the axis X in which the actuator is at the second insertion depth,
the actuator magnet exerting a magnetic field designed, when the locator is placed against the skin of a patient under which the valve has been implanted, the valve being placed in a window (26) of the locator, to unlock the rotor at the first insertion depth and to be rotationally coupled to the rotor at the second insertion depth.

2. Treatment device according to the immediately preceding claim, in which the guide is configured to guide the rotation of the actuator about the axis X from any angular position of the actuator about the axis X in which the actuator is at the second insertion depth, in a plane of rotation substantially perpendicular to the axis X.

3. Treatment device according to either one of the two immediately preceding claims, in which the difference between the first and second insertion depths, measured along the axis X, is greater than 5 millimetres.

4. Treatment device according to any one of the three immediately preceding claims, having first (96ₛ; 94ₛ; 92) and second (52, 54) stops defining the first and second insertion depths, respectively.

5. Treatment device according to any one of the four immediately preceding claims, in which the key has a riser (52) on which the actuator is mounted movably in translation along an axis Y of the key, which axis coincides with the axis X of the locator receptacle when the key is inserted into the locator receptacle.

6. Treatment device according to the immediately preceding claim, having a spring (54) interposed between the actuator and the riser or a spring (100) interposed between the actuator and the locator in such a way as to oppose an insertion of the actuator, along the axis Y, beyond the second insertion depth.

7. Treatment device according to the immediately preceding claim, in which said one spring extends along an axis parallel to the axis X and/or the axis Y or in a plane perpendicular to the axis X or to the axis Y.

8. Treatment device according to either one of the two immediately preceding claims, in which said spring is received in a receptacle and, at the side of the opening of the receptacle, is configured in such a way as to push a stub towards the axis X or towards the axis Y.

9. Treatment device according to any one of the four immediately preceding claims, in which the actuator is guided in translation on the riser, along the axis Y.

10. Treatment device according to any one of the nine immediately preceding claims, in which the actuator has a set of magnets (72) arranged in a Halbach configuration.

11. Treatment device according to any one of the six immediately preceding claims, in which the actuator has a support (56) to which said actuator magnet (72) is rigidly secured, a flange (59) mounted movably in translation, along the axis Y, with respect to the booster (52), and means (82; 85) to prevent any displacement of the support, along the axis Y, with respect to the flange, while permitting a displacement of the support perpendicularly to the axis Y, with respect to the flange.

12. Treatment device according to any one of the preceding claims, in which the actuator magnet is configured to exert a magnetic field designed, when the valve is in the open air and the locator is placed perpendicularly to the axis of rotation of the rotor of the valve,
- to unlock the rotor only when the actuator magnet is at a distance from the rotor of less than 20 mm; and/or
- to be rotationally coupled to the rotor only when the actuator magnet is at a distance from the rotor of less than 30 mm.
